# FASCICULE DE BREVET EUROPEEN

(11) **EP 2 739 262 B1**
(45) Date de publication et mention de la délivrance du brevet: **27.09.2017**
(21) Numéro de dépôt: 12750478.5
(22) Date de dépôt: 03.08.2012
(51) Int. Cl.: A61K 8/49, A61Q 19/02, A61K 31/343, A61K 31/415, A61K 31/416, A61K 31/44, A61P 17/00, A61P 17/02, A61P 35/00

(54) **UTILISATION D'ANTAGONISTE DU RÉCEPTEUR CB1 EN TANT QU'AGENT BLANCHISSANT ET/OU ANTI-BRUNISSEMENT DES MATIÈRES KÉRATINIQUES**
VERWENDUNG EINES CB1-REZEPTORANTAGONISTEN ALS AUFHELL- UND/ODER BRÄUNUNGSSCHUTZMITTEL FÜR KERATINMATERIAL
USE OF A CB1 RECEPTOR ANTAGONIST AS A WHITENING AND/OR ANTI-BROWNING AGENT FOR KERATIN MATERIAL

(30) Priorité: 05.08.2011 FR 1157214
(43) Date de publication de la demande: 11.06.2014
(73) Titulaire: L'OREAL, 75008 Paris (FR)
(72) Inventeur: JOURDAIN, Roland, F-92360 Meudon La Forêt (FR); ZUCCOLO, Michela, F-75011 Paris (FR); BRETON, Lionel, F-78000 Versailles (FR); MACCARRONE, Mauro, I-67100 L'aquila (IT)
(74) Mandataire: Pillet, Anne-Helene
(86) Numéro de dépôt international: PCT/FR2012/051847
(87) Numéro de publication internationale: WO 2013/021129

(56) Documents cités:
- EP-A1- 2 196 190
- EP-A2- 0 417 632
- US-A1- 2010 016 347
- US-A1- 2010 040 568
- RECEVEUR J M ET AL: "Conversion of 4-cyanomethyl-pyrazole-3-carboxamides into CB1 antagonists with lowered propensity to pass the blood@?brain-barrier", BIOORGANIC & MEDICINAL CHEMISTRY LETTERS, PERGAMON, ELSEVIER SCIENCE, GB, vol. 20, no. 2, 15 janvier 2010 (2010-01-15), pages 453-457, XP026812508, ISSN: 0960-894X [extrait le 2009-12-04] cité dans la demande
- SOFIA MAGINA ET AL: "Inhibition of basal and ultraviolet B-induced melanogenesis by cannabinoid CB1 receptors: a keratinocyte-dependent effect", ARCHIVES OF DERMATOLOGICAL RESEARCH, vol. 303, no. 3, 5 février 2011 (2011-02-05), pages 201-210, XP055026334, ISSN: 0340-3696, DOI: 10.1007/s00403-011-1126-z cité dans la demande
- C. BLAZQUEZ ET AL: "Cannabinoid receptors as novel targets for the treatment of melanoma", THE FASEB JOURNAL, vol. 20, no. 14, 1 décembre 2006 (2006-12-01), pages 2633-2635, XP055027753, ISSN: 0892-6638, DOI: 10.1096/fj.06-6638fje

## Description

La présente invention concerne l'utilisation cosmétique et/ou dermatologique d'antagonistes du récepteur CB1, dans une composition pour décolorer et/ou dépigmenter la peau et/ou les poils et/ou les cheveux.

La couleur des cheveux et de la peau humaine est fonction de différents facteurs et notamment des saisons de l'année, de l'origine ethnique, du sexe et de l'âge. Elle est principalement déterminée par la concentration, dans les kératinocytes, de la mélanine produite par les mélanocytes. Les mélanocytes sont les cellules spécialisées qui, par l'intermédiaire d'organelles particuliers, les mélanosomes, synthétisent la mélanine. Cette synthèse de mélanine, ou mélanogenèse, est le résultat de l'interaction entre le α-MSH (α-melanocyte stimulating hormone) et son récepteur MC1 R, interaction qui active une cascade de signalisation induisant l'enzyme tyrosinase *via* le facteur de transcription MITF.

La mélanogénèse est particulièrement complexe et fait intervenir schématiquement les principales étapes suivantes :

Tyrosine ---> Dopa ---> Dopaquinone ---> Dopachrome ---> Mélanine

La tyrosinase (monophénol dihydroxyl phénylalanine : oxygen oxydoreductase / EC 1,14,18,1) est l'enzyme essentielle intervenant dans cette suite de réactions. Elle catalyse notamment la réaction de transformation de la tyrosine en Dopa (dihydroxyphénylalanine) et la réaction de transformation de la Dopa en Dopaquinone.

Dans l'épiderme, le mélanocyte est impliqué dans l'unité mélanique épidermique qui comporte un mélanocyte entouré d'environ 36 kératinocytes voisins. Tous les individus, sans distinction de phototype, ont approximativement le même nombre de mélanocytes pour une zone cutanée donnée. Les différences ethniques, en terme de pigmentation, ne sont pas dues au nombre de mélanocytes, mais aux propriétés de leurs mélanosomes. Les mélanosomes sont agrégés en complexes et sont de petites tailles. Ce sont des organelles hautement spécialisées dont l'unique fonction est la production de mélanine. Ils naissent du réticulum endoplasmique sous formes de vacuoles sphériques appelées prémélanosomes. Les prémélanosomes contiennent un substrat protéinique amorphe, mais pas d'enzymes mélanogènes. Au cours de la maturation du prémélanosome, le substrat amorphe s'organise en une structure fibrillaire orientée dans l'axe longitudinal du mélanosome. On distingue quatre stades du développement du mélanosome correspondant à l'intensité de la mélanisation. La mélanine est déposée uniformément sur le réseau fibrillaire interne du mélanosome et l'opacité de l'organelle augmente jusqu'à saturation. Au fur et à mesure que la mélanine est synthétisée dans les mélanosomes, ceux-ci se déplacent de la région périnucléaire vers l'extrémité des dendrites des mélanocytes. Par phagocytose, l'extrémité des dendrites est capturée par les kératinocytes, les membranes dégradées et les mélanosomes redistribués dans les kératinocytes.

A différentes périodes de leur vie, certaines personnes voient apparaître sur la peau et plus spécialement sur les mains, des taches plus foncées et/ou plus colorées, conférant à la peau une hétérogénéité. Ces taches sont dues notamment à une concentration importante de mélanine dans les kératinocytes situés à la surface de la peau.

L'utilisation de substances dépigmentantes topiques inoffensives présentant une bonne efficacité est toute particulièrement recherchée en vue de traiter les taches pigmentaires.

L'arbutine, la niacinamide et l'acide kojique sont connus comme agents dépigmentants de la peau.

Alors que l'on pensait qu'il n'existait aucune autre voie de mélanogénèse qui ne dépende pas de α-MSH et de MC1 R, la Demanderesse a découvert, de façon surprenante, que l'activation du récepteur cannabinoide CB1 permet de stimuler la mélanogénèse et que des antagonistes du récepteur cannabinoïde CB1 ont la propriété d'empêcher les ligands naturels de ce récepteur (endocannabinoides) d'avoir un effet stimulateur de la mélanogénèse. En d'autres termes, la Demanderesse a pu démontrer que des antagonistes du récepteur cannabinoïde CB1 inhibaient l'effet pro-pigmentant des ligands naturels de ce récepteur. Les antagonistes du récepteur CB1 permettent ainsi d'inhiber la biosynthèse de la mélanine.

Les récepteurs CB1 et CB2 appartiennent à la superfamille des récepteurs transmembranaires, couplés aux protéines G. Le récepteur CB1 est codé par le gène CNR1 présent sur le chromosome 6. Les endocannabinoïdes sont des lipides actifs qui sont connus comme ligands de ces récepteurs CB1 et CB2. L'un des principaux endocannabinoïdes est l'anandamide ou AEA (N-arachidonoylethanolamine). La synthèse de l'AEA dépend de l'enzyme N-acyl-phosphatidylethanolamines (NAPE)-specific phospholipase D (NAPE-PLD), alors que sa dégradation a lieu par hydrolyse par l'enzyme FAAH (fatty acid amide hydrolase). Le méthanandamide ou mAEA (R(+)-arachidonyl-1'-hydroxy-2'-propylamide) est un analogue non hydrolysable de l'AEA et est, comme l'AEA, un ligand agoniste des récepteurs CB1, CB2 et TRPV1. L'entrée des endocannabinoïdes dans la cellule est saturable et énergie-dépendante, et elle a lieu via le transporteur membranaire d'endocannabinoïdes (EMT).

L'AEA et les protéines qui se lient à ce composé, le transportent, le synthétisent et l'hydrolysent font partie du système endocannabinoïde (ECS). Les endocannabinoïdes ont été identifiés - à la fois chez l'homme et chez la souris - dans un large nombre de types cellulaires de la peau et des structures associées, tels les kératinocytes épidermiques, les mastocytes du derme, les sébocytes et l'épithélium du follicule pileux (Maccarrone et al, J Biol Chem, 2003, Dobrosi et al., FASEB J, 2008). Il a été montré que les éléments du ECS contrôlent la prolifération, la différentiation, l'apoptose et la production de cytokines des cellules de la peau, ce qui suggère un rôle de ce système dans l'homéostasie de l'épiderme. Par ailleurs, la liaison de ligands agonistes comme l'AEA sur les récepteurs cannabinoïdes dans la peau entraine une atténuation des processus inflammatoires, tandis que des antagonistes aggravent des épisodes inflammatoires cutanés comme dans les pathologies de type dermatites allergiques.

Il est connu que certains antagonistes du récepteur CB1 peuvent permettre de réduire la prise alimentaire ou le poids de sujets obèses (Bioorganic & Medicinal Chemistry Letters 20 (2010), 453-457) ou peuvent aider au sevrage du tabac (J Pharmacol Exp Ther. 2005 Mar;312(3):875-83). Ces composés n'ont cependant jamais été décrits comme agissant sur des mélanocytes ou comme pouvant avoir un effet sur la production de mélanine comme dans la présente invention.

Magina et al. (Arch Dermatol Res. 2011) décrit une inhibition de la mélanogénèse suite à l'exposition de co-cultures de kératinocytes et de mélanocytes à un rayonnement UV. Les mélanocytes utilisés dans ces co-cultures sont particuliers puisque ce sont ici des cellules de mélanome humain, c'est-à-dire des cellules cancéreuses. Les auteurs émettent l'hypothèse que cet effet sur la mélanogénèse serait dû à l'action de médiateurs endocannabinoïdes secrétés par les kératinocytes et agissant sur les mélanocytes environnants. Il n'est cependant pas évoqué dans ce document, que l'utilisation d'un antagoniste du récepteur CB1 permettrait d'inhiber la mélanogénèse.

EP 2 196 190 A1, US 2010/016347 A1, US 2010/040568 A1 et EP 0 417 632 A2 divulguent l'utilisation de composés comme agents blanchissants et/ou anti-brunissements des matières kératiniques. Toutefois, aucun de ces documents ne décrit des antagonistes du récepteur CB1.

Ainsi, la présente invention a pour objet l'utilisation cosmétique non-thérapeutique d'au moins un antagoniste du récepteur CB1, en tant qu'agent blanchissant et/ou anti-brunissement des matières kératiniques. Un antagoniste du récepteur CB1 est, selon la présente invention, toute substance, composé simple ou complexe, d'origine naturelle ou synthétique qui s'oppose à l'activation de ce récepteur et qui s'oppose notamment à l'induction de la réponse biologique que l'on obtient avec le ligand naturel de ce récepteur (en particulier un endocannabinoïde tel que l'anandamide). Un antagoniste du récepteur CB1 peut agir ainsi soit en se fixant sur le récepteur cannabinoïde CB1 présent dans le corps humain, notamment dans la peau, et en s'opposant à son activation (on pourra alors parler de ligand antagoniste du récepteur CB1), soit en dégradant le ligand naturel de ce récepteur ou en diminuant ou inhibant la synthèse ou la libération endogène de ce ligand (on pourra alors parler d'antagoniste fonctionnel du récepteur CB1). En l'occurrence, un antagoniste de CB1 empêchera des endocannabinoïdes tels que l'anandamide ou certains de ces analogues comme le mAEA ou l'ACEA (Arachidonoyl 2'-chloroethylamide) d'induire une réponse biologique.

Au sens de la présente invention, une substance ou un composé qui « s'oppose » à l'activation d'un récepteur ou à l'induction d'une réponse biologique, est une substance ou un composé qui diminue ou inhibe partiellement ou totalement cette activation ou cette induction.

Préférentiellement, un antagoniste du récepteur CB1 selon l'invention est un ligand antagoniste du récepteur CB1 ou un antagoniste fonctionnel du récepteur CB1.

Le caractère antagoniste du récepteur CB1 d'une substance, composé simple ou complexe sera évalué par toute méthode connue de l'homme du métier, notamment par une méthode de mesure d'affinité réceptorielle sélective (dite de « binding ») couplée à une mesure de l'activité fonctionnelle dudit composé ou de ladite substance (voir notamment J. Med. Chem. 2007, 50, 3851-3856) ou par une méthode de libération contrôlée des endocannabinoïdes (dite méthode de « release »).

Par « matière kératinique », on entend la peau (du visage, du corps, du cuir chevelu) et les muqueuses, les cheveux, les cils, les sourcils et les ongles. Préférentiellement, on entend par « matière kératinique », la peau et/ou les muqueuses.

Par « agent blanchissant », on entend toute substance ou composé susceptible de blanchir les matières kératiniques et/ou de diminuer la pigmentation des matières kératiniques.

Par « agent anti-brunissement », on entend toute substance ou composé susceptible d'empêcher ou de réduire la coloration naturelle des matières kératiniques, c'est-à-dire la coloration naturelle « basale » ou induite par des évènements naturels (expositions aux UV, vieillissement chronologique...).

On entend par « coloration naturelle », la coloration ou la pigmentation de la peau résultant d'une exposition au soleil ou aux UVA et/ou UVB.

L'antagoniste du récepteur CB1 tel que défini ci-dessus est préférentiellement choisi parmi le 5-(4-Chlorophenyl)-1-(2,4-dichloro-phenyl)-4-methyl-*N*-(piperidin-1-yl)-1*H*-pyrazole-3-carboxamide (également connu sous les noms de SR1, de SR141716 ou de rimonabant ou sous le numéro CAS 158681-13-1 et notamment commercialisé par la société Sigma Aldrich ou sous la marque Acomplia™ ou Zimulti™ par la société Sanofi Aventis), le TM38837 (développé par la société 7TM Pharma), le 1-(2,4-dichlorophenyl)-5-(4-iodophenyl)-4-methyl-N-(1-piperidyl)pyrazole-3-carboxamide (connu sous le code AM251 et le numéro CAS 183232-66-8), le 4S-(-)-3-(4-chlorophenyl)-N-methyl-N'-[(4-chlorophenyl)-sulfonyl]-4-phenyl-4,5-dihydro-1H-pyrazole-1-carboxamidine (connu sous le nom Ibipinabant, les codes SLV319 et BMS-646,256 et le numéro CAS 464213-10-3), le 4-[6-methoxy-2-(4-methoxyphenyl)1-benzofuran-3-carbonyl]benzonitrile (connu sous le code LY-320,135 et le numéro CAS 176977-56-3 et développé par la société Eli Lilly), le N-((2S,3S)-3-(3-cyanophenyl)-4-(4-ethoxyphenyl)butan-2-yl)-2-methyl-2-(5-methylpyridin-2-yloxy)propanamide (connu sous le code MK-9470 et le numéro CAS 945850-36-2), le 8-chloro-1-(2,4-dichlorophenyl)-1,4,5,6-tetrahydro-N-1-piperidinyl-benzo[6,7]cyclohepta[1,2-c]pyrazole-3-carboxamide (connu sous le code NESS-0327 et le numéro CAS 494844-07-4), le 5-(4-bromophenyl)-1-(2,4-dichlorophenyl)-4-ethyl- N-(1-piperidinyl)-1H-pyrazole-3-carboxamide (connu sous les noms Surinabant ou SR147778 et le numéro CAS 288104-79-0 et développé par la société Sanofi Aventis), le N-[(1S,2S)-3-(4-Chlorophenyl)-2- (3-cyanophenyl)-1-methylpropyl]-2-methyl-2- ((5-(trifluoromethyl)pyridin-2-yl)oxy)propanamide (connu sous les noms Taranabant ou MK-0364 et le numéro CAS 701977-09-5 et découvert par la société Merck) et le 5-(4-chlorophenyl)- 3-[(E)-2-cyclohexylethenyl]- 1-(2,4-dichlorophenyl)- 4-methyl- 1H-pyrazole (connu sous le nom VCHSR).

Tous ces composés sont des antagonistes connus du récepteur CB1 (voir notamment les documents J Med Chem 1999, 42:769-776 ; Journal of medicinal chemistry 47 (3):627-43 ; Journal of Pharmacology and Experimental Therapeutics 1998; 284(1):291-7; Proc. Natl. Acad. Sci. U.S.A. 104 (23):9800-5 ; Journal of Pharmacology and Experimental Therapeutics 2003, 306(1):363-70 ; Journal of Pharmacology and Experimental Therapeutics 2004 Sep, 310(3):905-14 et Journal of Pharmacology and Experimental Therapeutics 2007, 321(3):1013-22).

Le blanchiment ou blanchissement de la peau ou la diminution de sa pigmentation peuvent être appréciés visuellement ou par des méthodes de mesure physique de la coloration de la peau ou des cheveux, bien connues de l'homme du métier (notamment par le système de mesure colorimétrique (L*, a*, b*)).

L'antagoniste du récepteur CB1 tel que défini ci-dessus, peut être présent dans une composition à une concentration comprise entre 0,00001% à 10% en poids, de préférence entre 0,001% à 5% en poids, notamment entre 0,01 à 1% en poids, par rapport au poids total de la composition.

Préférentiellement, l'antagoniste du récepteur CB1, tel que défini ci-dessus, est utilisé comme agent de blanchissement de la peau et/ou des muqueuses et/ou comme agent pour diminuer la pigmentation de la peau et/ou des muqueuses et/ou pour inhiber la mélanogénèse, de préférence par l'intermédiaire d'une inactivation des récepteurs CB1 ou de la dégradation de leurs ligands naturels.

L'invention décrit aussi un antagoniste du récepteur CB1, tel que défini ci-dessus, pour son utilisation dans le traitement d'un désordre hyperpigmentaire choisi parmi le mélasma, le chloasma, les lentigines, le lentigo sénile, les taches de rousseur, les hyperpigmentations post-inflammatoires dues à une abrasion et/ou une brûlure et/ou une cicatrice et/ou une dermatose et/ou une allergie de contact; les nevi, les hyperpigmentations à déterminisme génétique, les hyperpigmentations d'origine métabolique ou médicamenteuse et les mélanomes. Par le terme « traitement », on désigne aussi bien la prévention, la diminution ou l'atténuation.

Le lentigo sénile, également dénommé lentigo actinique ou solaire, tache sénile, tache de vieillesse, ou encore communément dénommé « crasse sénile », « marguerites de cimetière » ou « fleurs de cimetière », est de loin la plus fréquente des lésions pigmentaires. Sa présence s'accentue avec l'âge des individus, et se manifeste tout particulièrement sur des zones de la peau qui ont été photo-exposées, telles que le visage, le dos des mains, les membres supérieurs et notamment la face dorsale des avant-bras, le dos, et en particulier le haut du dos.

Avantageusement, une utilisation selon l'invention permet en particulier d'effacer les lentigos séniles en régulant et harmonisant, au sens de répartition homogène, la distribution des mélanocytes dans la lame basale dermo-épidermique. Le mélasma (ou chloasma) est constitué par des nappes pigmentées s'étendant de façon presque toujours symétrique sur le front, sur les joues et sur la lèvre supérieure. Elle recouvre les pommettes et parfois les tempes en respectant habituellement l'extrémité du nez, les paupières et le menton. De petites taches lenticulaires sont souvent disséminées entre les nappes principales. Il peut être aggravé et parfois déclenché par l'exposition solaire.

Les désordres hyperpigmentaires englobent aussi les situations d'hyperpigmentation consécutive à une inflammation. L'hyperpigmentation post-inflammatoire est plutôt indépendant du degré d'inflammation et dépend plus de la nature du trauma ayant provoqué l'inflammation. L'hyperpigmentation post-inflammatoire peut être sévère après certaines lésions comme les brûlures thermiques. L'hyperpigmentation post-inflammatoire peut persister des mois, voire des années.

La composition pour l'utilisation selon l'invention est en particulier adaptée à une application topique ou adaptée à une administration orale et contient par ailleurs un milieu physiologiquement acceptable. Les antagonistes du récepteur CB1 selon l'invention, seuls ou en mélange, ainsi que la composition les comprenant, peuvent être utilisés en application topique sur les matières kératiniques ou en administration orale. La composition peut être notamment une composition cosmétique ou une composition dermatologique.

Par « adaptée à une application topique » ou « adaptée à une administration orale », on entend respectivement « convenant à une application topique » ou « convenant à une administration orale ».

Par « milieu physiologiquement acceptable », on entend, selon l'invention, un milieu cosmétiquement ou pharmaceutiquement acceptable compatible avec la peau (y compris le cuir chevelu), les cheveux, les muqueuses et/ou les yeux.

Par « milieu cosmétiquement acceptable », on entend un milieu sans odeur ou aspect désagréable, et qui ne génère pas de picotement, de tiraillement ou de rougeur inacceptable pour l'utilisateur, lors de son application topique sur la peau ou ses annexes.

Le milieu physiologiquement acceptable sera adapté à la nature du support sur lequel doit être appliqué la composition ainsi qu'à la forme sous laquelle la composition est destinée à être conditionnée, notamment solide ou fluide à température ambiante et pression atmosphérique.

La composition peut également comprendre tous les additifs cosmétiques usuels tels que l'eau, les solvants, les huiles, les cires, les pigments, les charges, les tensioactifs, les actifs cosmétiques ou dermatologiques, les filtres UV, les polymères, les gélifiants, les conservateurs et les parfums.

Bien entendu, l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition selon l'invention peut se présenter sous toutes les formes galéniques normalement utilisées dans les domaines cosmétiques et dermatologiques; elle peut être notamment sous forme d'une solution aqueuse, hydroalcoolique ou huileuse, éventuellement gélifiée, d'une dispersion du type lotion éventuellement biphasée, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Un autre objet de l'invention est un procédé cosmétique non-thérapeutique de blanchissement de la peau et/ou des muqueuses comprenant l'application topique sur la peau et/ou les muqueuses ou l'administration orale, d'une composition comprenant, dans un milieu physiologiquement acceptable, un antagoniste du récepteur CB1 tel que défini ci-dessus.

Préférentiellement, ce procédé est un procédé de dépigmentation de la peau et/ou des muqueuses.

Dans ce procédé, l'antagoniste du récepteur CB1 peut être présent à une concentration comprise entre à 0,00001 et 10% en poids par rapport au poids total de la composition, de préférence entre 0,001% à 5% en poids, notamment entre 0,01 à 1% en poids.

Ce procédé cosmétique peut comprendre une application ou une administration unique. Selon un mode de réalisation particulier, l'application ou l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

La présente demande décrit également un procédé de sélection d'agent blanchissant et/ou anti-brunissement des matières kératiniques comprenant :
a) une étape de pré-sélection d'un antagoniste du récepteur CB1,
b) une étape de sélection finale d'un antagoniste du récepteur CB1 en tant qu'agent blanchissant et/ou anti-brunissement, lorsque la quantité de mélanine produite par des mélanocytes mis en contact avec ledit antagoniste est égale ou significativement inférieure à celle produite par des mélanocytes n'ayant pas été en contact avec cet antagoniste du récepteur CB1.

Un « antagoniste du récepteur CB1 » est tel que défini ci-dessus.

Le caractère antagoniste du récepteur CB1, d'un produit sera évalué par toute méthode connue de l'homme du métier, notamment par une méthode de mesure d'affinité réceptorielle sélective (dite de « binding ») couplée à une mesure de l'activité fonctionnelle dudit composé ou de ladite substance (voir notamment J. Med. Chem. 2007, 50, 3851-3856) ou par une méthode de libération contrôlée des endocannabinoïdes (dite méthodes de « release »).

La mesure d'affinité réceptorielle sélective peut être réalisée en mettant en contact des récepteurs CB1 (ou des membranes de cellules comprenant ces récepteurs) avec des ligands marqués (e.g. des ligands radiomarqués, immunofluorescents...), en éliminant les ligands marqués qui ne se sont pas liés aux récepteurs et en déterminant la quantité de ligands marqués qui se sont liés aux récepteurs.

La mesure de l'activité fonctionnelle d'un composé ou d'une substance pour valider son caractère antagoniste du récepteur CB1 peut être réalisée en mesurant la quantité d'AMP cyclique (AMPc) produite par une culture cellulaire (i.e. une culture de mélanocytes ou une coculture de mélanocytes et de kératinocytes ou un épiderme reconstruit pigmenfe ou un explant de peau), suite à l'ajout de forskoline et dudit composé ou de ladite substance. Si aucune augmentation de la quantité d'AMPc n'est observée suite à l'ajout du composé ou de la substance alors son caractère antagoniste du récepteur CB1 est validé. L'AMP cyclique est en effet l'un des composés connus dont la synthèse est inhibée suite à l'activation du récepteur CB1. La forskoline est connue comme augmentant la synthèse d'AMPc.

Une méthode de libération contrôlée des endocannabinoides se fera par mise en contact d'un composé ou d'une substance avec une culture cellulaire (i.e. une culture de mélanocytes ou une coculture de mélanocytes et de kératinocytes ou un épiderme reconstruit pigmenté ou un explant de peau) et par mesure de la quantité d'endocannabinoides produite.

Dans l'étape b) la quantité de mélanine produite par des mélanocytes peut être mesurée sur une culture de mélanocytes ou sur une coculture de mélanocytes et de kératinocytes ou sur un épiderme reconstruit pigmenté ou sur un explant de peau. A l'étape b), l'antagoniste du récepteur CB1 présélectionné à l'étape a) peut être ajouté à une concentration déterminée sur l'une ou l'autre de ces cultures de cellules et la quantité de mélanine produite par les mélanocytes présents dans ces cultures est mesurée. Une mesure contrôle est effectuée sur l'une ou l'autre de ces cultures de cellules sans avoir ajouté d'antagoniste du récepteur CB1.

De manière optionnelle, à l'étape b), les cultures cellulaires mentionnées ci-dessus peuvent, préalablement à la mesure de la quantité de mélanine produite par les mélanocytes, être soumises à un stress et notamment à une exposition à des UVA et/ou UVB.

La sélection finale d'un antagoniste du récepteur CB1 en tant qu'agent blanchissant et/ou anti-brunissement des matières kératiniques à l'étape b) du procédé de sélection est faite par comparaison de la quantité de mélanine produite par des mélanocytes mis en contact avec ledit antagoniste du récepteur CB1 et de la quantité de mélanine produite par des mélanocytes n'ayant pas été mis en contact avec ledit antagoniste du récepteur CB1. Un antagoniste du récepteur CB1 peut être ainsi sélectionné en tant qu'actif diminuant la pigmentation des matières kératiniques, s'il diminue d'au moins 10%, préférentiellement d'au moins 20%, encore plus préférentiellement d'au moins 30%, la quantité de mélanine produite par des mélanocytes n'ayant pas été en contact avec ledit antagoniste du récepteur CB1.

Les conditions de culture des mélanocytes seuls ou en coculture avec des kératinocytes ainsi que les méthodes de préparation d'épiderme reconstruit pigmenté (voir notamment EP 1 878 790) sont connues de l'homme du métier.

La mesure de la quantité de mélanine produite par les mélanocytes, éventuellement en co-culture avec des kératinocytes ou présents dans l'épiderme reconstruit pigmenté, est faite selon des méthodes connues de l'homme du métier (voir par exemple Xiao et al., Arch Dermatol Res 2007 ; 299: 245-57). Préférentiellement, cette mesure est réalisée par spectrophotométrie et encore plus préférentiellement par spectrophotométrie à 450 nm.

Ce procédé de sélection, permet de sélectionner spécifiquement des composés ou des substances qui peuvent être utilisés comme agents pour blanchir ou dépigmenter la peau et/ou des poils et/ou des cheveux. Ces produits ont la particularité d'inhiber ou de réduire la mélanogénèse ou la pigmentation de la peau et/ou des poils et/ou des cheveux, par l'intermédiaire de l'inactivation du récepteur CB1.

Dans la description et dans les exemples suivants, sauf indication contraire, les pourcentages sont des pourcentages en poids et les plages de valeurs libellées sous la forme "entre ... et ..." incluent les bornes inférieure et supérieure précisées. Les ingrédients sont mélangés, avant leur mise en forme, dans l'ordre et dans des conditions facilement déterminées par l'homme de l'art.

Légende des figures :
Figure 1 : Le SR141716 (Rimonabant) solubilisé dans DMSO a été évalué en application systémique à 0.1 et 0.5µM sur ERP non irradiés. Les évaluations sont faites contre placebo (DMSO) et en comparaison à l'actif dépigmentant de référence, le lucinol.
Figure 2 : Le SR141716 (Rimonabant) solubilisé dans DMSO a été évalué en application systémique à 0.1 et 0.5µM sur ERP irradiés UV. Les évaluations sont faites contre placebo (DMSO) et en comparaison à l'actif dépigmentant de référence, le lucinol.

### Exemple 1 : Mise en évidence de l'effet dépigmentant d'antagonistes du récepteur CB1

### 1. Matériels et méthodes

### 1.1. Composés testés

Anandamide (AEA) et methanandamide (mAEA) sont fournis par Sigma Chemical Co. (St. Louis, MO).

### 1.2. Réactifs utilisés

Le composé SR11 = *N*-Piperidino-5-(4-chlorophenyl)-1-(2,4-dichloro-phenyl)-4-methyl-3-pyrazole carboxamide (antagoniste spécifique du récepteur CB1) est fournis par Sanofi-Aventis Recherche (Montpellier, France).

### 1.3. Culture cellulaire et traitement

Des mélanocytes épidermiques humains normaux (NHEM) isolés de prépuce et fournis par Promocell (Heidelberg, Allemagne), sont cultivés pendant 24h à 37°C dans une atmosphère humide à 5% de CO₂ dans un milieu de culture adapté M2 (Promocell).

L'AEA et les autres composés sont ajoutés directement dans le milieu de culture. 24h après chaque traitement, la viabilité des cellules a été mesurée par l'utilisation du colorant d'exclusion bleu Trypan (Maccarone et al., 2003).

Le mAEA et l'AEA sont testés *in vitro* à une concentration de 1 µM et comparés. Le mAEA est aussi utilisé à cette concentration quand il est utilisé en combinaison avec le SR1 à 0,1 µM.

### 1.4. Détermination du contenu en mélanine des mélanocytes

La quantité en mélanine présente dans les cellules NHEM est mesurée selon une méthode connue de l'homme du métier (Xiao et al., Arch Dermatol Res 2007 ; 299 : 245-57). Selon cette méthode, les cellules NHEM sont traitées à la trypsine et lavées deux fois avec un tampon phosphate salin. Ensuite les échantillons sont resuspendus dans 200 µl d'eau MilliQ et 1 ml d'un mélange éthanol : éther (1:1, vol/vol) est ajouté pour éliminer les substances opaques autres que la mélanine. Après 15 minutes, les échantillons sont centrifugés à 600g pendant 5 minutes. Le culot est séché à l'air et dissout dans 200 µl de soude 1N. Les échantillons sont ensuite chauffés à 80°C pendant une heure et refroidis.

La quantité de mélanine est déterminée par spectrophotométrie à 450 nm en utilisant les contrôles standards de mélanine fournis par Sigma Chemical Co., de façon à vérifier la gamme de linéarité de l'essai. La quantité de mélanine est exprimée par rapport au contrôle.

### 1.5. Analyse statistique

Les résultats donnés sont les moyennes avec leurs écarts type de 3 essais indépendants, chacun réalisé en triplicata. Ils ont été comparés par analyse de variance (ANOVA).

### 2. Résultats

### 2.1. Démonstration de l'effet dépigmentant des composés testés

| | **Contrôle** | **mAEA***** | **AEA***** | **mAEA+SR1**^{**##**#} |
|---|---|---|---|---|
| **Moyenne** | 1,10 | 2,80 | 2,09 | 1,22 |
| **Ecart-type (SD)** | 0,10 | 0,26 | 0,22 | 0,09 |
| **Erreur Standard (SEM)** | 0,06 | 0,15 | 0,13 | 0,05 |

| | | | | |
|---|---|---|---|---|
| ***p<0.001 vs contrôle | | | | |

L'anandamide et son analogue mAEA augmentent de manière significative la quantité de mélanine dans les mélanocytes (NHEM) par l'intermédiaire du récepteur CB1.

L'utilisation du SR1, antagoniste connu du récepteur CB1 empêche le mAEA d'avoir ses effets pro-pigmentants et donc permet d'inhiber la pigmentation de la peau.

### 2.2. Conclusions

Compte tenu de ces résultats, le composé SR1 peut donc être utilisé comme un agent blanchissant ou anti-brunissement de la peau.

### Exemple 2 : Compositions cosmétiques

### Crème dépigmentante ou blanchissante

| **Ingrédients** | **Quantités (en gramme)** |
|---|---|
| SR1 | 5,00 |
| Ceteareth 30 | 7,00 |
| Stéarate de glycéryle | 2,00 |
| Alcool cétylique | 1,50 |
| Polydiméthylsiloxane | 1,50 |
| Huile de paraffine | 15,00 |
| Glycérine | 20,00 |
| Conservateurs | q.s. |
| Eau déminéralisée | qsp 100,00 g |

Après application de cette crème sur la peau, on observe une diminution de la pigmentation de la peau.

### Exemple 3 : Evaluation de l'effet dépigmentant sur epidermes reconstruits pigmentés (ERP)

Des épidermes pigmentés sont reconstruits sur un support collagénique (BPER) à l'aide de mélanocytes et kératinocytes humains normaux.

Les épidermes sont obtenus après 8 jours de reconstruction, les mélanocytes choisis sont de phototype estimé IV.1

Certains épidermes sont traités quotidiennement par le placebo, d'autres par le produit à tester SR14716 à différentes concentrations (0,1 µM et 0,5µM).

Le produit à tester est évalué à deux concentrations en comparaison à son solvant seul (placebo) et à un actif dépigmentant de référence, le lucinol à 500µM. Les épidermes reconstruits ainsi traités sont irradiés ou non avec des UV.²

Chaque condition expérimentale est réalisée en quadruplicat.

La qualité histologique des épidermes après traitement est évaluée sur coupes histologiques après coloration HES afin de déterminer l'impact du traitement sur les épidermes. Aussi la physiologie des mélanocytes est étudiée pour détecter une éventuelle mélano-cytotoxicité. Si aucune altération histologique ni aucun effet mélano-toxique du produit évalué n'est révélé, une quantification de la pigmentation est effectuée par quantification de la mélanine sur coupes histologiques par analyse d'images

### Analyse des résultats

Une quantification de la pigmentation par quantification microscopique de la mélanine est alors réalisée et permet d'objectiver une activité propigmentante du composé à tester.

La mélanine présente dans l'épiderme est colorée sur coupes histologiques (coloration Fontana Masson) puis quantifiée par analyse d'images. Pour cela, chaque épiderme est photographié sur toute sa largeur à l'aide d'un microscope. Environ 10 images sont acquises par épiderme (lumière blanche, grossissement X20).

La surface occupée par la mélanine est quantifiée à l'aide d'un logiciel d'analyse d'image développé à façon. Brièvement, chaque image est segmentée en 4 zones selon leur couleur (lumière, stratum corneum, épiderme vivant et BPER). La mélanine présente dans l'épiderme sous forme de grains noirs est seuillée comme étant la zone la plus sombre de l'épiderme sur le plan rouge. Les pixels occupés par la mélanine dans l'épiderme entier ou les couches vivantes sont ensuite quantifiés.

Cette quantification rend compte de la surface occupée par la mélanine ainsi que de la localisation moyenne de la mélanine dans l'épiderme.

Les résultats ci-dessous sont exprimés en pourcentage de stimulation relative par rapport au témoin cultivé en milieu de co-culture standard.

### Sans irradiation UV :

**Tableau 1**

| composé testé | % | valeur |
|---|---|---|
| DMSO 1/1000 SYST D17 | 100 | 10,5263158 |
| Lucinol 30µM SYST D17 | 10,5263158 | 1,31578947 |
| SR141716 0,1µM SYST D17 | 64,4736842 | 6,57894737 |
| SR141716 0,5µM SYST D17 | 72,3684211 | 5,26315789 |

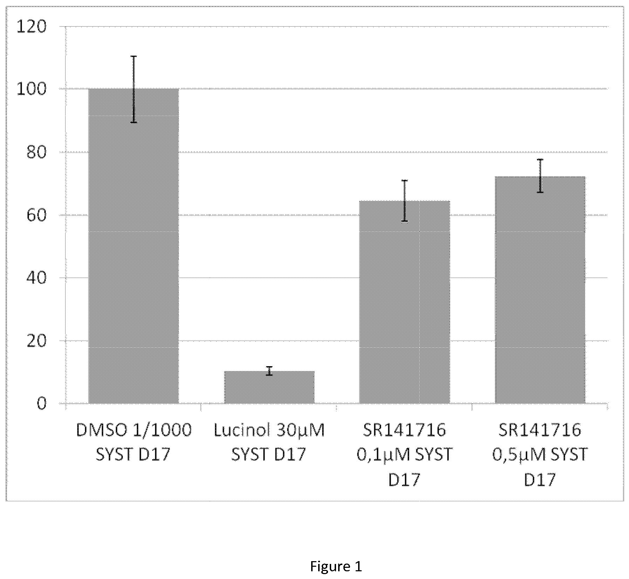

Les résultats sont présentés dans la figure 1.

On observe que le SR141716 induit une diminution importante de la production de mélanine dans les épidermes pigmentés reconstruits, de manière dose-dépendante.

### Avec irradiation UV :

**Tableau 2**

| composé testé | % | valeur |
|---|---|---|
| DMSO 1/1000 SYST D15 | 100 | 10,6382979 |
| DMSO 1/1000 150mJ/cm² UVB SYST D15 | 131,914894 | 12,7659574 |
| Lucinol 50µM 150mJ/cm² UVB SYST D15 | 31,9148936 | 4,25531915 |
| SR141716 0,1µM 150mJ/cm² UVB SYST D15 | 76,5957447 | 8,5106383 |
| SR141716 0,5µM 150mJ/cm² UVB SYST D15 | 55,3191489 | 8,5106383 |

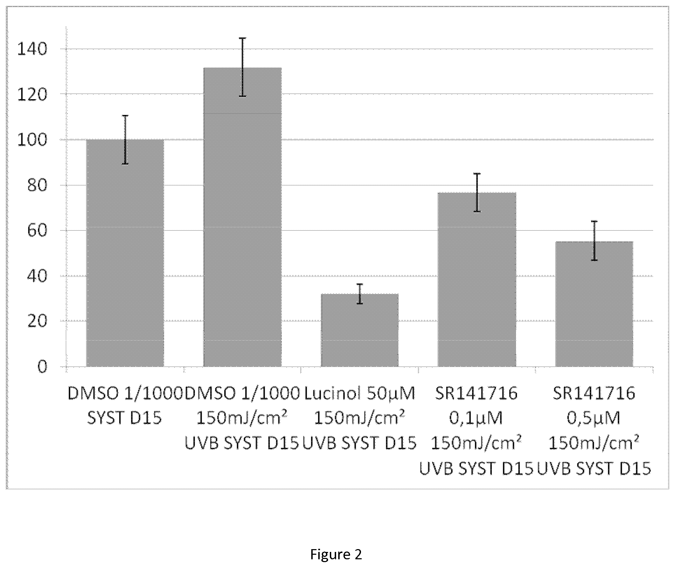

Les résultats sont présentés dans la figure 2.

On observe que le SR141716 induit une diminution importante de la production de mélanine dans les épidermes pigmentés reconstruits, de manière dose-dépendante.

## Revendications

1. Utilisation cosmétique non-thérapeutique d'au moins un antagoniste du récepteur CB1, en tant qu'agent blanchissant et/ou anti-brunissement des matières kératiniques,

2. Utilisation selon la revendication précédente, **caractérisée en ce que** ledit antagoniste est choisi parmi le 5-(4-Chlorophenyl)-1-(2,4-dichloro-phenyl)-4-methyl-*N*-(piperidin-1-yl)-1*H*-pyrazole-3-carboxamide, , le 1-(2,4-dichiorophenyl)-5-(4-iodophenyl)-4-methyl-N-(1-piperidyl)pyrazole-3-carboxamide, le 4S-(-)-3-(4-chlorophenyl)-N-methyl-N'-[(4-chlorophenyl)-sulfonyl]-4-phenyl-4,5-dihydro-1H-pyrazole-1-carboxamidine, le 4-[6-methoxy-2-(4-methoxyphenyl)1-benzofuran-3-carbonyl]benzonitrile, le N-((2S,3S)-3-(3-cyanophenyl)-4-(4-ethoxyphenyl)butan-2-yl)-2-methyl-2-(5-methylpyridin-2-yloxy)propanamide, le 8-chloro-1-(2,4-dichlorophenyl)-1,4,5,6-tetrahydro-N-1-piperidinyl-berizo[6,7]cyclohepta[1,2-c]pyrazole-3-carboxamide, le 5-(4-bromophenyl)-1-(2,4-dichlorophenyl)-4-ethyl- N-(1-piperidinyl)-1H-pyrazole-3-carboxamide, le N-[(1S,2S)-3-(4-Chlorophenyl)-2- (3-cyanophenyl)-1-methylpropyl]-2-methyl-2- ((5-(trifluoromethyl)pyridin-2-yl)oxy)propanamide et le 5-(4-chlorophenyl)- 3-[(E)-2-cyclohexyiethenyl]- 1-(2,4-dichlorophenyl)- 4-methyl- 1H-pyrazole.

3. Utilisation selon l'une quelconque des revendications 1 ou 2, comme agent de blanchissement de la peau et/ou des muqueuses.

4. Utilisation selon l'une quelconque des revendications 1 à 3, comme agent pour diminuer la pigmentation de la peau et/ou des muqueuses.

5. Utilisation selon l'une quelconque des revendications précédentes, comme agent pour inhiber la mélanogénèse.

6. Procédé cosmétique non-thérapeutique de blanchissement de la peau et/ou des muqueuses comprenant l'application topique sur la peau et/ou les muqueuses ou l'administration orale, d'une composition comprenant, dans un milieu physiologiquement acceptable, un antagoniste du récepteur CB1 tel que défini dans l'une quelconque des revendications 1 à 2.

7. Procédé selon la revendication 6, **caractérisé en ce que** ledit antagoniste du récepteur CB1 est présent à une concentration comprise entre à 0,00001 et 10% en poids par rapport au poids total de la composition.

## Patentansprüche

1. Nichttherapeutische kosmetische Verwendung von mindestens einem CB1-Rezeptor-Antagonisten als Aufhellmittel und/oder Bräunungsschutzmittel für Keratinmaterial.

2. Verwendung nach dem vorhergehenden Anspruch, **dadurch gekennzeichnet, dass** der Antagonist aus 5-(4-Chlorphenyl)-1-(2,4-dichlorphenyl)-4-methyl-N-(piperidin-1-yl)-1*H*-pyrazol-3-carboxamid, 1-(2,4-Dichlorphenyl)-5-(4-iodphenyl)-4-methyl-N-(1-piperidyl)pyrazol-3-carboxamid, 4S-(-)-3-(4-Chlorphenyl)-N-methyl-N'[(4-Chlorphenyl)sulfonyl]-4-phenyl-4,5-dihydro-1H-pyrazol-1-carboxamid, 4-[6-Methoxy-2-(4-methoxyphenyl)-1-benzofuran-3-carbonyl]benzonitril, N-((2S,3S)-3-(3-Cyanophenyl)-4-(4-ethoxyphenyl)butan-2-yl)-2-methyl-2-(5-methylpyridin-2-yloxy)propanamid, 8-Chlor-1-(2,4-dichlorphenyl)-1,4,5,6-tetrahydro-N-1-piperidinylbenzo[6,7]cyclohepta[1,2-c]pyrazol-3-carboxamid, 5-(4-Bromphenyl)-1-(2,4-dichlorphenyl)-4-ethyl-N-(1-piperidinyl)-1H-pyrazol-3-carboxamid, N-[(1S,2S)-3-(4-Chlorphenyl)-2-(3-cyanophenyl)-1-methylpropyl]-2-methyl-2-((5-(trifluormethyl)pyridin-2-yl)oxy)propanamid und 5-(4-Chlorphenyl)-3-[(E)-2-cyclohexylethenyl]-1-(2,4-dichlorphenyl)-4-methyl-1H-pyrazol ausgewählt ist.

3. Verwendung nach einem der Ansprüche 1 oder 2 als Aufhellmittel für die Haut und/oder die Schleimhäute.

4. Verwendung nach einem der Ansprüche 1 bis 3 als Mittel zur Reduktion der Pigmentierung der Haut und/oder der Schleimhäute.

5. Verwendung nach einem der vorhergehenden Ansprüche als Mittel zum Hemmen der Melanogenese.

6. Nichttherapeutisches kosmetisches Verfahren zum Aufhellen der Haut und/oder der Schleimhäute, umfassend die topische Applikation auf die Haut und/oder die Schleimhäute oder die orale Verabreichung einer Zusammensetzung, die einen CB1-Rezeptor-Antagonisten wie in einem der Ansprüche 1 bis 2 definiert in einem physiologisch unbedenklichen Medium umfasst.

7. Verfahren nach Anspruch 6, **dadurch gekennzeichnet, dass** der CB1-Rezeptor-Antagonist in einer Konzentration zwischen 0,00001 und 10 Gew.-% in Bezug auf das Gesamtgewicht der Zusammensetzung vorliegt.

## Claims

1. Nontherapeutic cosmetic use of at least one CB1 receptor antagonist as a whitening and/or anti-browning agent for keratin materials.

2. Use according to the preceding claim, **characterized in that** said antagonist is chosen from 5-(4-chlorophenyl)-1-(2,4-dichlorophenyl)-4-methyl-*N*-(piperidin-1-yl)-1*H*-pyrazole-3-carboxamide, 1-(2,4-dichlorophenyl)-5-(4-iodophenyl)-4-methyl-N-(1-piperidyl)pyrazole-3-carboxamide, 4S-(-)-3-(4-chlorophenyl)-N-methyl-N'-[(4-chlorophenyl)sulfonyl]-4-phenyl-4,5-dihydro-1H-pyrazole-1-carboxamidine, 4-[6-methoxy-2-(4-methoxyphenyl)-1-benzofuran-3-carbonyl]benzonitrile, N-((2S,3S)-3-(3-cyanophenyl)-4-(4-ethoxyphenyl)butan-2-yl)-2-methyl-2-(5-methylpyridin-2-yloxy)propanamide, 8-chloro-1-(2,4-dichlorophenyl)-1,4,5,6-tetrahydro-N-1-piperidinylbenzo[6,7]cyclohepta[1,2-c]pyrazole-3-carboxamide, 5-(4-bromophenyl)-1-(2,4-dichlorophenyl)-4-ethyl-N-(1-piperidinyl)-1H-pyrazole-3-carboxamide, N-[(1S,2S)-3-(4-chlorophenyl)-2-(3-cyanophenyl)-1-methylpropyl]-2-methyl-2-((5-(trifluoromethyl)pyridin-2-yl)oxy)propanamide and 5-(4-chlorophenyl)-3-[(E)-2-cyclohexylethenyl]-1-(2,4-dichlorophenyl)-4-methyl-1H-pyrazole.

3. Use according to either one of Claims 1 and 2, as a whitening agent for the skin and/or the mucous membranes.

4. Use according to any one of Claims 1 to 3, as an agent for reducing the pigmentation of the skin and/or of the mucous membranes.

5. Use according to any one of the preceding claims, as an agent for inhibiting melanogenesis.

6. Nontherapeutic cosmetic process for whitening the skin and/or the mucous membranes, comprising the topical application to the skin and/or the mucous membranes, or the oral administration, of a composition comprising, in a physiologically acceptable medium, a CB1 receptor antagonist as defined in either one of Claims 1 and 2.

7. Process according to Claim 6, **characterized in that** said CB1 receptor antagonist is present at a concentration of between 0.00001% and 10% by weight, relative to the total weight of the composition.
